# EUROPEAN PATENT APPLICATION

(11) **EP 1 484 008 A1**
(43) Date of publication of application: **08.12.2004**
(21) Application number: 04012512.2
(22) Date of filing: 27.05.2004
(51) Int. Cl.: A61B 5/00

(54) **Non-invasive method of determinining an analyte concentration using a hyperosmotic solution**

(30) Priority: 02.06.2003 US 474839 P
(71) Applicant: Bayer HealthCare, LLC, Tarrytown, New York 10591 (US)
(72) Inventor: Dosmann, Andrew J., Granger, Indiana 46530 (US)
(74) Representative: Linhart, Angela, Dr.

(57) **Abstract**

A non-invasive method of determining the concentration of an analyte comprises topographically applying a hyperosmotic solution to an area of the skin. The hyperosmotic solution is adapted to at least partially absorb into the area of the skin such that the skin becomes generally transparent. An optical readhead is placed over the generally transparent area of the skin. The amount of light of the analyte is measured using at least one wavelength via the optical readhead. The concentration of the analyte is calculated from the amount of light. The analyte may be glucose and the hyperosmotic solution may be glycerol.

## Description

### FIELD OF THE INVENTION

The present invention relates to methods of detecting analyte concentrations and, more specifically, methods of detecting analyte concentration such as glucose in a non-invasive manner using hyperosmotic fluids.

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, determining glucose in body fluids is important to diabetic individuals who must frequently check the glucose level in their body fluids to regulate the glucose intake in their diets. Determining the glucose concentration may be done in an invasive or non-invasive manner. Since invasive methods generally involve drawing a fluid such as blood with a lancet, it would be desirable to have a reliable non-invasive glucose monitoring technique.

One of the most significant barriers to non-invasive glucose monitoring i s that water in the skin absorbs 99% of the light. Thus, the determination of glucose includes a water background that makes the glucose measurement much more difficult and unreliable because of the noise level associated with this background. Additionally, the skin scatters the light which makes the skin look nearly opaque to an optical readhead. More specifically, the water, collagen and other molecules in the skin scatter most of the light which makes the skin look nearly opaque to an optical readhead. To attempt to overcome these problems, a method to improve the reduction of noise level has used an intense near-infrared (NIR) light source to measure the transmission and/or reflectance at many wavelengths throughout the NIR. This method has several drawbacks, however, since it requires expensive equipment and extensive patient calibration scenarios that make the method impractical.

It would be desirable to provide a method that detects an analyte concentration such as glucose in a non-invasive manner that overcomes the above-noted shortcomings.

### SUMMARY OF THE INVENTION

According to one non-invasive method, the concentration of an analyte is determined and comprises topographically applying a hyperosmotic solution to an area of the skin. The hyperosmotic solution is adapted to at least partially absorb into the area of the skin such that the skin becomes generally transparent. An optical readhead is placed over the generally transparent area of the skin. T he amount o flight of the a nalyte is measured using at least one wavelength via the optical readhead. The concentration of the analyte is calculated from the amount of light.

According to another non-invasive method, the concentration of glucose comprises topographically applying a hyperosmotic solution to an area of the skin. The hyperosmotic solution is adapted to at least partially absorb into the area of the skin such that the skin becomes generally transparent. An optical readhead is placed over the generally transparent area of the skin. The amount of light of glucose is measured using at least one wavelength via the optical readhead. The concentration of glucose is calculated from the amount of light.

According to a further non-invasive method, the concentration of glucose comprises topographically applying glycerol to an area of the skin such that the skin becomes generally transparent. An optical readhead is placed over the generally transparent area of the skin. The amount of light of glucose is measured using at least one mid-infrared wavelength, near-infrared wavelength or a combination thereof via the optical readhead. The concentration of glucose is calculated from the amount of light.

According to one non-invasive method, the concentration of an analyte is calculated comprising topographically applying a hyperosmotic solution to an area of the skin. The hyperosmotic solution is adapted to at least partially absorb into the area of the skin such that the skin becomes generally transparent. An optical readhead is placed over the generally transparent area of the skin. The amount of light of the analyte is measured using at least one wavelength via the optical readhead.

### BRIEF DESCRIPTION OF THE DRAWINGS

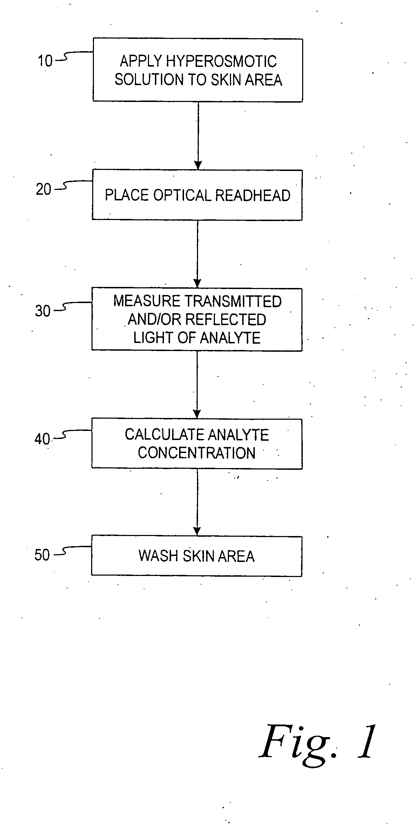

FIGURE 1 is a flowchart according to one method of the present invention.

While the invention is susceptible to various modifications and alternative forms, specific methods thereof have been shown by way of example in the drawing and will herein be described in detail. It should be understood, however, that it is not intended to limit the invention to the particular forms disclosed but, on the contrary, the intention is to cover all modifications, equivalents, and alternatives falling within the spirit and scope of the invention as defined by the appended claims.

### DETAILED DESCRIPTION OF THE ILLUSTRATED EMBODIMENT

The present invention is directed to non-invasive methods of determining the concentration of an analyte. In one method of the present invention, the analyte is glucose. It is contemplated, however, that other analytes may be measured. For example, it is contemplated that the non-invasive methods of the present invention may be used to determine the concentration of cholesterol, albumin, or fructose. It is contemplated that the non-invasive methods of the present invention may be used to determine the concentration of other analytes such as lactate or bilirubin. The present invention is not limited, however, to these specific analytes.

According to one non-invasive method, the analyte concentration is determined by topographically applying a hyperosmotic solution to an area of the human skin. This is shown in step 10 of FIGURE 1. It is contemplated that the present invention may be used with other skin, such as animal skin. The hyperosmotic solution is adapted to at least partially absorb into the area of the skin such that the skin becomes generally transparent. It is desirable for the hyperosmotic solution to be adapted to substantially or fully absorb into the area of the skin such that the skin becomes substantially or fully transparent. The hyperosmotic solution is adapted to at least partially replace the water of the area of the skin. This is desirable because water, collagen and other molecules in the skin contribute to background noise by scattering most of the light. It is believed that the ability to see deeper into the tissue of the skin significant reduces the background absorbance.

One example of a hyperosmotic solution that may be used is glycerol. Glycerol is desirable as a hyperosmotic solution because its refraction index matches the refraction index of collagen better than that of water, resulting in the light being allowed to pass through the area of the skin. The use of glycerol as the hyperosmotic solution allows the skin to become substantially or fully transparent.

An optical readhead is placed over the generally transparent area of the skin as shown in step 20 of FIGURE 1. The optical readhead may be a reflective readhead, a transmissive readhead, or a combination thereof. The transmitted and/or reflected light of the analyte is measured at at least one selected wavelength. The optical readhead is configured to measure transmitted and/or reflected light of the analyte. The optical readhead also includes a light source such as a conventional low-cost light emitting diode (LED).

One example of an optical readhead that may be used in the non-invasive method of the present invention is an optical readhead that is used commercially to measure blood oxygen levels using pulse oximetry. Such an optical readhead used in pulse oximetry measures transmitted light. Such an optical readhead would likely need to be modified by one skilled in the art to measure the exact selected analyte. For example, if the analyte to be measured is glucose in blood, then one skilled in the art would select a specific wavelength(s) in the optical readhead to measure the glucose. One method of modifying the optical readhead is to select an LED at a near infrared wavelength where there is a known glucose absorbance band. The LED would be used to illuminate the sample through the skin, and the reflected or transmitted light would be detected. The detector might be modified from, for example, a standard silicon detector to a lead sulfide d etector. T he s ilicon detector h as a p hotosensitivity in t he visible wavelength region, while the lead sulfide detector has photosensitivity in the infrared region.

One commercial example of an optical readhead that may be used in the present invention is manufactured by Philips Medical Systems-Medical Supplies (3000 Minuteman Rd., MS 0040 Andover, Massachusetts 01810, United States). Depending on the analyte to be measured, the optical readhead would likely need to be modified by one skilled in the art.

Alternatively, reflective spectrophotometry may be used to measure the reflected light of the analyte at at least one selected wavelength. Another method that may be used involves Fourier Transform Infrared Spectrophotometry (FTIR) which is based on reflected light but has different detection optics in comparison to reflectance spectroscopy.

Referring still to FIGURE 1, the transmitted and/or reflected light of the analyte is measured at at least one wavelength via the optical readhead in step 30. It is desirable to measure the transmitted and/or reflected light of the analyte at a mid-infrared frequency (from about 1.5 - 25 micrometers (µm)). It is contemplated, however, that the transmitted and/or reflected light of the analyte may be measured at wavelengths other than mid-infrared wavelengths or combinations using mid-infrared wavelengths. For example, the transmitted and/or reflected light may be measured at a near-infrared (NIR) wavelength, which is defined herein as being from about 0.90 to about 2.0 micrometers (µm). The transmitted and/or reflected light of the analyte may be measured at a plurality of wavelengths (e.g., a plurality of mid- and/or near-infrared wavelengths). Depending on the analyte, it may be desirable to measure at a plurality of wavelengths because other analytes may absorb at similar frequencies. Thus, measuring at a plurality of wavelengths may improve the reliability and accuracy of the measurements.

It is especially preferable to measure the transmitted and/or reflected light of glucose at a plurality of mid-infrared wavelengths because it is believed that glucose absorbance is strongest at such wavelengths. It is contemplated that other wavelengths may be used such as near-infrared wavelengths. Some selected wavelengths that may be used to measure the transmitted and/or reflected light of glucose are from about 1 to about 15 micrometers and, more specifically, about 1.9 micrometers. Such wavelengths are believed to correlate well with the measuring of glucose concentrations.

The analyte concentration from the amount of light is calculated in step 40 of FIGURE 1. In one method of calculating the concentration of the analyte (e.g., glucose), the amount of light at selected wavelength(s) are correlated with known glucose concentrations. Thus, an unknown glucose concentration can be determined using the amount of reflective and/or transmitted light at selected wavelength(s). Such a calculation may render periodic patient calibration unnecessary. It is contemplated that there are many methods of correlating the absorbance of one or more wavelengths to the glucose concentration. According to one method, a glucose calibration algorithm is built. One example of such a glucose algorithm is disclosed in Provisional Application No. 60/355,358 entitled "Non-Invasive System for the Determination of Analytes in Body Fluids" that was filed on February 11, 2002, which is hereby incorporated by reference in its entirety.

In this glucose calibration algorithm, spectral data is obtained from the body tissue of at least a first and second test subject which is combined to generate a model useful for predicting the glucose levels for all of the subjects contributing data. The raw signals of the test subjects are normalized by checking for outliers by standard methods known in the art and further preprocessing by Orthogonal Signal Correction (OSC) reduction and wavelets analysis filtering to enhance the glucose signal and to suppress the water and other background signals. The resulting set of spectra is then used to build a calibration model by partial least squares (PLS) regression using Venetian blinds cross-validation on at least a portion of the data. It is contemplated that other data preparation techniques may be used to reduce or remove the background signal including, but not limited to, first-derivative smoothing, second-derivative smoothing, wavelength selection by means of genetic algorithms, wavelet processing and principal component analysis. The calibration models may be generated by other techniques such as different forms of regression, including principal components regression, ridge regression or ordinary (inverse) least squares regression.

As shown in optional step 50, the area of the skin may be washed with a solvent (e.g., water) to substantially remove the hyperosmotic solution and to hydrate the area of skin.

While particular embodiments and applications of the present invention have been illustrated and described, it is to be understood that the invention is not limited to the precise methods disclosed herein and that various modifications, changes, and variations may be apparent from the foregoing descriptions without departing from the spirit and scope of the invention as defined in the appended claims.

## Claims

1. A non-invasive method of determining the concentration of an analyte comprising:
topographically applying a hyperosmotic solution to an area of the skin, the hyperosmotic solution being adapted to at least partially absorb into the area of the skin such that the skin becomes generally transparent;
placing an optical readhead over the generally transparent area of the skin;
measuring the amount of light of the analyte using at least one wavelength via the optical readhead; and
calculating the concentration of the analyte from the amount of light.

2. The method of claim 1, wherein the analyte is glucose, cholesterol, albumin, or fructose.

3. The method of claim 1, wherein the at least one selected wavelength is at a mid-infrared wavelength, or is at near-infrared wavelength.

4. The method of claim 1, wherein measuring of the amount of reflected light of the analyte occurs at a plurality of selected wavelengths.

5. The method of claim 1, wherein the measured amount of light is transmitted light.

6. The method of claim 1, wherein the measured amount of light is reflected light.

7. The method of claim 1, wherein the skin is human.

8. A non-invasive method of determining the concentration of glucose comprising:
topographically applying a hyperosmotic solution to an area of the skin, the hyperosmotic solution being adapted to at least partially absorb into the area of the skin such that the skin becomes generally transparent;
placing an optical readhead over the generally transparent area of the skin;
measuring the amount of light of the glucose using at least one wavelength via the optical readhead; and
calculating the concentration of glucose from the amount of light.

9. The method of claim 8, wherein the at least one selected wavelength is at a mid-infrared wavelength.

10. The method of claim 8, wherein the at least one selected wavelength is at a near-infrared wavelength.

11. The method of claim 8, wherein measuring of the amount of light of the analyte occurs at a plurality of selected wavelengths.

12. The method of claim 8, wherein the measured amount of light is reflected light.

13. The method of claim 8, wherein the measured amount of light is transmitted light.

14. The method of claim 8, wherein the skin is human skin.

15. A non-invasive method used in calculating the concentration of an analyte comprising:
topographically applying a hyperosmotic solution to an area of the skin, the hyperosmotic solution being adapted to at least partially absorb into the area of the skin such that the skin becomes generally transparent;
placing an optical readhead over the generally transparent area of the skin; and
measuring the amount of light of the analyte using at least one wavelength via the optical readhead.
